(19) Europäisches Patentamt
European Patent Office
Office européen des brevets

(11) Veröffentlichungsnummer: **0 385 224 B1**

# (12) EUROPÄISCHE PATENTSCHRIFT

(45) Veröffentlichungstag der Patentschrift: **15.12.93**

(51) Int. Cl.[5]: **C07D 231/12**, C07D 231/16, C07D 231/56, A01N 43/56

(21) Anmeldenummer: **90103176.5**

(22) Anmeldetag: **20.02.90**

Die Akte enthält technische Angaben, die nach dem Eingang der Anmeldung eingereicht wurden und die nicht in dieser Patentschrift enthalten sind.

## (54) Substituierte N-Hydroxypyrazole und Fungizide, die diese Verbindungen enthalten.

(30) Priorität: **25.02.89 DE 3905948**

(43) Veröffentlichungstag der Anmeldung:
**05.09.90 Patentblatt 90/36**

(45) Bekanntmachung des Hinweises auf die Patenterteilung:
**15.12.93 Patentblatt 93/50**

(84) Benannte Vertragsstaaten:
**AT BE CH DE DK ES FR GB GR IT LI NL SE**

(56) Entgegenhaltungen:
**EP-A- 0 160 804**
**EP-A- 0 226 917**
**EP-A- 0 253 213**

(73) Patentinhaber: **BASF Aktiengesellschaft**
**Carl-Bosch-Strasse 38**
**D-67063 Ludwigshafen(DE)**

(72) Erfinder: **Schuetz, Franz, Dr.**
**Budapester Strasse 45**
**D-6700 Ludwigshafen(DE)**
Erfinder: **Sauter, Hubert, Dr.**
**Neckarpromenade 20**
**D-6800 Mannheim 1(DE)**
Erfinder: **Brand, Siegbert, Dr.**
**Hegelstrasse 39**
**D-6940 Weinheim(DE)**
Erfinder: **Wenderoth, Bernd, Dr.**
**Schwalbenstrasse 26**
**D-6840 Lampertheim(DE)**
Erfinder: **Baus, Ulf, Dr.**
**Keltenweg 10**
**D-6915 Dossenheim(DE)**
Erfinder: **Reuther, Wolfgang, Dr.**
**Am Pferchelhang 16**
**D-6900 Heidelberg(DE)**
Erfinder: **Lorenz, Gisela, Dr.**
**Erlenweg 13**
**D-6730 Neustadt(DE)**
Erfinder: **Ammermann, Eberhard, Dr.**
**Sachsenstrasse 3**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue substituierte N-Hydroxypyrazole und Fungizide, welche diese Verbindungen enthalten.

Es ist bekannt, Acrylsäuremethylester, z.B. den $\alpha$-(2-Phenoxymethyl-phenyl)-$\beta$-methoxy-acrylsäuremethylester als Fungizide zu verwenden (DE-A-35 45 319, EP-A-226 917). Seine fungizide Wirkung ist jedoch unbefriedigend. Es ist ferner bekannt, 1-Hydroxypyrazolderivate als Nitrifikationshemmer zu verwenden (EP-A-160 804). Auch die Verwendung von substituierten Phenylglyoxylsäuremethylester-0-methyloximen als Fungizide ist bekannt (EP-A-253 213).

Es wurde nun gefunden, daß substituierte N-Hydroxypyrazole der allgemeinen Formel I,

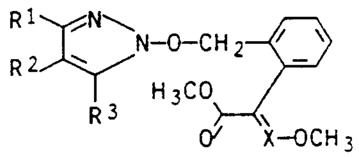

I

in der

$R^1$, $R^2$ und $R^3$ gleich oder verschieden sind und Wasserstoff, $C_1$-$C_4$-Alkyl, $C_1$-$C_2$-Halogenalkyl, $C_3$-$C_6$-Cycloalkyl, $C_1$-$C_4$-Alkoxycarbonyl, Halogen,Phenyl oder Phenyl-$C_1$-$C_4$-alkyl bedeuten, wobei der Phenylrest gebenenfalls durch einen oder mehrere der folgenden Reste substituiert ist: $C_1$-$C_4$-Alkyl, $C_3$-$C_6$-Cycloalkyl, $C_1$-$C_2$-Halogenalkyl, $C_1$-$C_4$-Alkoxy, Halogen, Cyano, Nitro,

oder $R^2$ und $R^3$ zusammen mit dem Pyrazolring, dessen Substituenten sie sind, einen Indazolring oder Tetrahydroindazolring bilden, wobei diese Ringe gegebenenfalls durch $C_1$-$C_4$-Alkyl substituiert sind,

X CH oder N bedeutet, und ihre pflanzenverträglichen Säureadditionssalze und Metallkomplexe,

eine ausgezeichnete fungizide wirkung besitzen, die besser ist, als die der bekannten Acrylsäuremethylester.

Die in der allgemeinen Formel aufgeführten Reste können beispielsweise folgende Bedeutung haben:

$R^1$, $R^2$ und $R^3$ sind gleich oder verschieden und bedeuten z.B. $C_1$-$C_4$-Alkyl (z.B. Methyl, Ethyl, n- oder iso-Propyl, n-, iso-, sec.- oder tert.-Butyl), $C_1$-$C_2$-Halogenalkyl (z.B. Difluormethyl, Trifluormethyl, Chlormethyl, Dichlormethyl, Trichlormethyl, Pentafluorethyl), $C_3$-$C_6$-Cycloalkyl (z.B. Cyclopropyl, Cyclobutyl, Cyclopentyl, Cyclohexyl), $C_1$-$C_4$-Alkoxycarbonyl (z.B. Methoxycarbonyl, Ethoxycarbonyl, Propoxycarbonyl, Butoxycarbonyl), Halogen (z.B. Fluor, Chlor, Brom, Jod) oder bedeuten Phenyl oder Phenyl-$C_1$-$C_4$-alkyl (z.B. Benzyl, 2-Phenylethyl, 3-Phenylpropyl, 4-Phenylbutyl), wobei der Phenylrest gegebenenfalls durch einen oder mehrere (einen bis drei) der folgenden Reste substituiert ist: $C_1$-$C_4$-Alkyl (z.B. Methyl, Ethyl, Propyl, Butyl), $C_3$-$C_6$-Cycloalkyl (z.B. Cyclopropyl, Cyclobutyl, Cyclopentyl, Cyclohexyl), $C_1$-$C_2$-Halogenalkyl (z.B. Trifluormethyl), $C_1$-$C_4$-Alkoxy (z.B. Methoxy, Ethoxy, n- oder iso-Propoxy, n-, iso-, sec.- oder tert.-Butoxy), Halogen (z.B. Fluor, Chlor, Brom, Jod), Cyano, Nitro oder $R^2$ und $R^3$ zusammen mit dem Pyrazolring, dessen Substituenten sie sind, einen Indazolring oder Tetrahydroindazolring bilden, wobei diese Ringe gegebenenfalls einfach bis dreifach durch $C_1$-$C_4$-Alkyl (z.B. Methyl, Ethyl, n- oder iso-Propyl, n-, iso-, sec.- oder tert.-Butyl) substituiert sind,

X kann CH oder N sein.

Salze sind beispielsweise die pflanzenverträglichen Säureadditionssalze, z.B. die Salze mit anorganischen oder organischen Säuren, wie beispielsweise die Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions im allgemeinen beliebig ist.

Ferner lassen sich die Verbindungen der Formel I nach bekannten Methoden in Metallkomplexe überführen. Das kann durch Umsetzung dieser Verbindungen mit Metallsalzen, z.B. Salzen der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die neuen Verbindungen der allgemeinen Formel I können bei der Herstellung aufgrund der C=C- bzw. C=N-Doppelbindung als E/Z-Isomerengemische anfallen. Diese können in der üblichen Weise, z.B. durch Kristallisation oder Chromatographie, in die einzelnen Komponenten getrennt werden. Sowohl die einzelnen isomeren Verbindungen als auch ihre Gemische werden von der Erfindung umfaßt und sind als Fungizide brauchbar.

Die Herstellung der neuen Verbindungen der allgemeinen Formel I gemäß Anspruch 1, erfolgt beispielsweise so, daß man die N-Hydroxypyrazole der allgemeinen Formel II zunächst mit einer Base (z.B. Natriumhydroxid oder Kaliumhydroxid) in die entsprechenden Natrium-oder Kaliumsalze überführt und diese dann in einem inerten Lösungs- oder Verdünnungsmittel mit einer substituierten Benzylverbindung der allgemeinen Formel III umsetzt.

Als Lösungs- oder Verdünnungsmittel kommen z.B. Aceton, Acetonitril, Dimethylsulfoxid, Dioxan, Dimethylformamid, N-Methyl-pyrrolidon, N,N'-Dimethylpropylenharnstoff oder Pyridin in Betracht. Außerdem kann es von Vorteil sein, der Reaktionsmischung einen Katalysator wie z.B. Tetramethylethylendiamin oder Tris-(3,6-dioxoheptyl)-amin in einer Menge von 0,01 bis 10 (Gew.-%), bezogen auf Verbindung III, zuzusetzen.

Die entsprechenden Umsetzungen können auch im Zweiphasensystem (z.B. Tetrachlorkohlenstoff/Wasser) durchgeführt werden. Als Phasentransferkatalysatoren kommen z.B. Trioctylpropylammoniumchlorid oder Cetyltrimethylammoniumchlorid in Betracht.

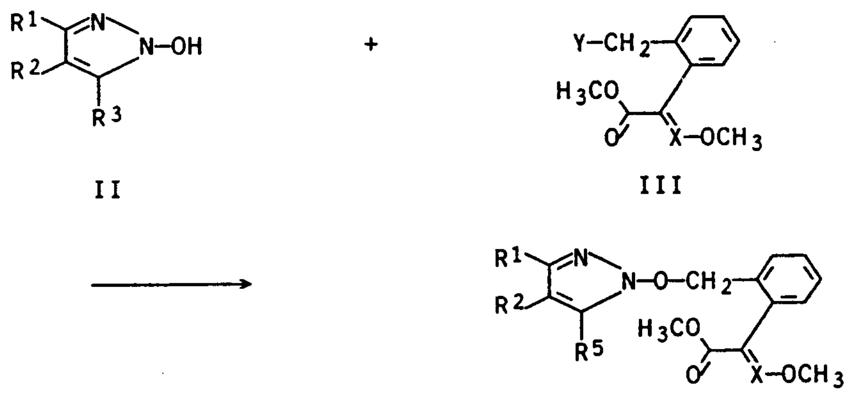
II
III

$R^1$, $R^2$, $R^3$ und X haben die oben genannten Bedeutungen, Y bedeutet Chlorid, Bromid, p-Toluolsulfonat, Methansulfonat, Trifluormethansulfonat.

Zur Darstellung der als Ausgangsverbindungen benötigten N-Hydroxypyrazole der Formel II werden Pyrazole der allgemeinen Formel IV zunächst in an sich bekannter Weise mit einem Alkalihydroxid, Alkalihydrid oder Alkalicarbonat in ihre Metallsalze der allgemeinen Formel V ($Me^+$ bedeutet ein Kation der Alkalimetalle) überführt. Die erhaltenen Metallsalze der Formel V werden anschließend in einem inerten organischen Lösungsmittel (z.B. Tetrahydrofuran) oder in einem Zweiphasensystem (z.B. Toluol/Wasser) gegebenenfalls in Gegenwart eines Phasentransferkatalysators (z.B. Benzyltriethylammoniumchlorid) mit Dibenzoylperoxid umgesetzt. Die Reaktionstemperatur liegt zwischen 0 und 60 °C.

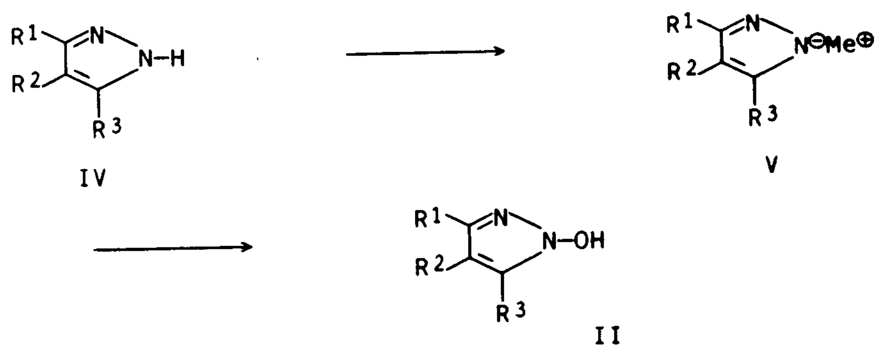
IV
V
II

Alternativ kann auch so vorgegangen werden, daß man ein Alkalimetallsalz der allgemeinen Formel V mit einer aliphatischen oder aromatischen Peroxocarbonsäure so umsetzt, daß die Reaktionstemperatur zwischen -5 °C und 60 °C liegt. Die Umsetzung kann in Wasser als Lösungsmittel oder in einem Zweiphasensystem aus Wasser und einem mit Wasser nur schlecht mischbaren inerten organischen Lösungsmittel (z.B. Toluol), gegebenenfalls in Gegenwart eines geeigneten Phasentransferkatalysators (z.B. Benzyltriethylammoniumchlorid) durchgeführt werden. Die Peroxocarbonsäure kann vor der Reaktion im Reaktionsgemisch aus $H_2O_2$ und einem Carbonsäurehalogenid oder einem Carbonsäureanhydrid hergestellt werden

oder in Form eines Alkali-oder Erdalkalisalzes eingesetzt werden.

Zur Darstellung der neuen Verbindungen der allgemeinen Formel I werden ferner die substituierten Benzylverbindungen der allgemeinen Formel III benötigt. Verbindungen der allgemeinen Formel IIIa (X = N, Y = Chlorid, Bromid) erhält man durch Halogenierung des 2-Methyl-phenylglyoxylsäure-methylester-O-methyloxims VI nach literaturbekannten Methoden. Dies erreicht man zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder durch Umsetzung mit N-Chlor-bzw. N-Brom-succinimid (vgl. Horner, Winkelmann, Angew. Chem. 71 (1959) 349).

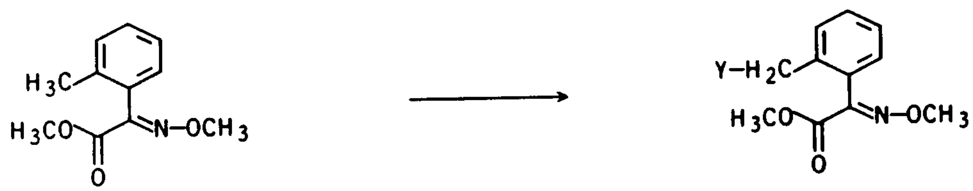

VI

IIIa: Y = Chlorid, Bromid

2-Methyl-phenylglyoxylsäure-methylester-O-methyloxim VI läßt sich herstellen, indem man 2-Methylphenylglyoxylsäure-methylester VII z.B. a) mit O-Methylhydroxylamin-hydrochlorid umsetzt oder b) mit Hydroxylamin-hydrochloridzum entsprechenden Oxim umsetzt und dieses danach mit einem Methylierungsmittel der Formel $CH_3$-L, in der L eine Abgangsgruppe (z.B. Chlorid, Bromid, Jodid, Methylsulfat) bedeutet, zur Reaktion bringt (vgl. DE-36 23 921).

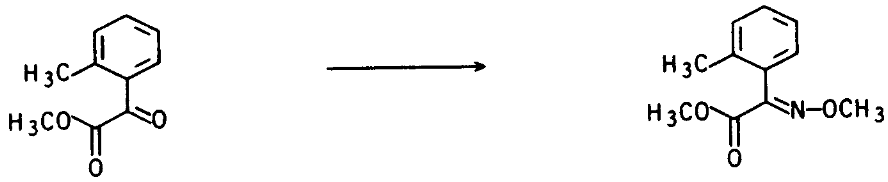

VII

VI

Benzylhalogenide der allgemeinen Formel IIIa (X = N, Y = Chlorid, Bromid) erhält man auch, wenn man 2-Halogenmethyl-phenylglyoxylsäuremethylester der Formel VIII (Hal = Chlorid, Bromid) a) mit O-Methylhydroxylamin-hydrochlorid umsetzt oder b) mit Hydroxylamin-hydrochlorid zum entsprechenden Oxim umsetzt und dieses mit einem Methylierungsmittel der Formel $CH_3$-L, in der L eine Abgangsgruppe (z.B. Chlorid, Bromid, Jodid, Methylsulfat) bedeutet, zur Reaktion bringt (vgl. DE-36 23 921).

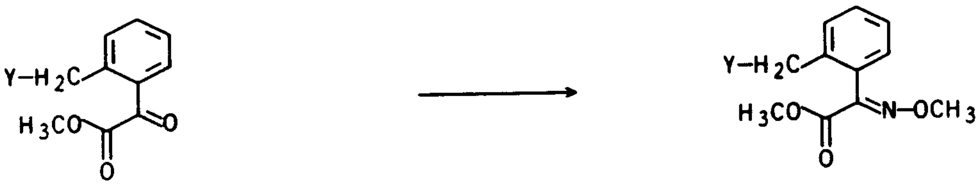

VIII

Y = Chlorid, Bromid

IIIa

2-Halogenmethyl-phenylglyoxylsäure-methylester der Formel VIII (Hal = Chlorid, Bromid) lassen sich herstellen, indem man 2-Methyl-phenylglyoxylsäuremethylester VII nach literaturbekannten Methoden halogeniert. Die Umsetzung erfolgt zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder mit N-Chlor- bzw. N-Brom-Succinimid (vgl. Horner, Winkelmann, Angew. Chem. 71 (1959) 349).

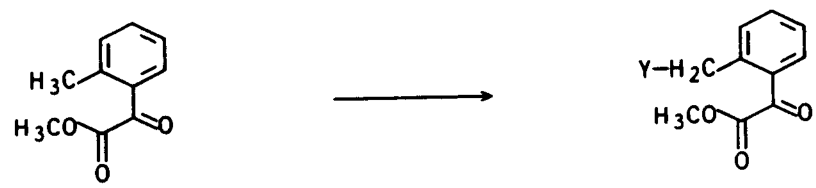

VII

VIII

Y = Chlorid, Bromid

Substituierte Benzylverbindungen der allgemeinen Formel IIIb (X = CH, Y = Chlorid, Bromid) sind entweder bekannt oder können nach bekannten Verfahren hergestellt werden. Entsprechende Herstellverfahren sind z.B. beschrieben in DE-35 19 280, DE-35 45 318 und DE-35 45 319.

Substituierte Benzylverbindungen der allgemeinen Formel IIIc (X = CH oder N, Y = p-Toluolsulfonat, Methansulfonat, Trifluormethansulfonat) lassen sich aus den entsprechenden Verbindungen der allgemeinen Formel IIIa (X = N, Y = Chlorid, Bromid) oder IIIb (X = CH, Y = Chlorid, Bromid) durch Umsetzung mit p-Toluolsulfonsäure (Y = p-Toluolsulfonat), Methansulfonsäure (Y = Methansulfonat) oder Trifluormethansulfonsäure (Y = Trifluormethansulfonat) herstellen. Die Reaktionen können z.B. in einem inerten Lösungs- oder Verdünnungsmittel (z.B. Dimethylformamid) in Gegenwart einer Base (z.B. Kaliumcarbonat) durchgeführt werden. Alternativ kann auch so vorgegangen werden, daß man die entsprechende Sulfonsäure zunächst in ihr Natrium- oder Kaliumsalz überführt und dieses dann in einem inerten Lösungs- oder Verdünnungsmittel (z.B. Dimethylformamid) mit einer Verbindung der allgemeinen Formel IIIa oder IIIb zu den substituierten Benzylverbindungen der allgemeinen Formel IIIc umsetzt.

## Beispiele

Die folgenden Beispiele und Vorschriften sollen die Herstellung der neuen Wirkstoffe und ihrer Vorprodukte erläutern.

### Vorschrift 1

2-(Brommethyl)-phenylglyoxylsäuremethylester-O-methyloxim

27,5 g (0,133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 %) der oben genannten Verbindung als Öl erhalten.

### Beispiel 1

2-[(3′,5′-Dimethyl-pyrazol-1′-yl)-oxymethyl]-phenylglyoxylsäure-methylester-O-methyloxim (Verbindung Nr. 28)

a) 28,8 g (0,30 mol) 3,5-Dimethylpyrazol werden in 500 ml Tetrahydrofuran gelöst und bei Raumtemperatur in kleinen Portionen mit 9,0 g (0,30 mol) einer 80 %igen Suspension von Natriumhydrid in Paraffinöl versetzt. Nach beendeter Wasserstoffentwicklung wird die Mischung auf 5 °C abgekühlt und mit 24,2 g (0,1 mol) trockenem Dibenzoylperoxid, gelöst in 500 ml Tetrahydrofuran, so versetzt, daß die Temperatur der Reaktionsmischung nicht über 25 °C steigt. Nach beendeter Zugabe wird noch zehn Minuten gerührt und die Reaktionsmischung mit Eiswasser und Petrolether versetzt und geschüttelt. Die wäßrige Phase wird abgetrennt und mit Schwefelsäure angesäuert. Man extrahiert zunächst mehrfach mit Cyclohexan und anschließend mit Essigsäureethylester. Die vereinigten Essigsäureethylesterphasen werden mit Natriumsulfat getrocknet und eingeengt. Man erhält so 8,4 g (25 %) 3,5-Dimethyl-1-hydroxypyrazol (Fp.: 155 °C).

b) Zu einer Lösung von 1,2 g (0,02 mol) Kaliumhydroxid in 50 ml Ethanol werden 2,2 g (0,02 mol) 3,5-Dimethyl-1-hydroxypyrazol in 30 ml Ethanol getropft. Man rührt zwei Stunden bei Raumtemperatur und engt das Reaktionsgemisch ein. Der Rückstand wird in 60 ml Dimethylformamid aufgenommen und mit

5,7 g (0,02 mol) 2-(Brommethyl)-phenylglyoxylsäuremethylester-O-methyloxim in 30 ml Dimethylformamid versetzt. Man rührt zwei Stunden bei 100 °C, zieht das Lösungsmittel im Vakuum ab und nimmt den Rückstand in Methyl-tert.-butylether auf. Die organische Phase wird mit Wasser gewaschen, getrocknet und eingeengt. Man erhält 3,7 g (59 der Titelverbindung als Öl (Verbindung Nr. 28).

Beispiel 2

α-2-[(4′-Chlor-pyrazo-1′-yl)-oxymethyl]phenyl-β-methoxy-acrylsäuremethylester (Verbindung Nr. 9)

a) 4,1 g (0,04 mol) 4-Chlorpyrazol werden in 16,8 g (0,15 mol) einer 50 %igen wäßrigen KOH gelöst. Die Lösung wird unter Rühren auf 0 °C abgekühlt und langsam mit 3,4 g (0,05 mol) einer 50 %igen wäßrigen Wasserstoffperoxid-Lösung versetzt. Danach werden portionsweise 7,4 g (0,05 mol) Phthalsäureanhydrid hinzugefügt und anschließend die Reaktionsmischung auf 20 °C erwärmt. Man rührt noch mehrere Stunden, erwärmt zur Zersetzung des Peroxids noch kurz auf 80 °C und kühlt das Reaktionsgemisch wieder auf Raumtemperatur (20 °C) ab. Danach säuert man mit Schwefelsäure an, filtriert das ausgefallene Kaliumsulfat ab und extrahiert mit Essigsäureethylester. Die vereinigten organischen Phasen werden getrocknet und eingeengt. Der Rückstand wird mit Cyclohexan überschichtet und kristallisiert. Man erhält so 3,2 g (68 %) 4-Chlor-1-hydroxypyrazol (Fp.: 125 °C).
b) Zu einer Lösung von 1,2 g (0,02 mol) Kaliumhydroxid in 50 ml Ethanol werden 2,4 g (0,02 mol) 4-Chlor-1-hydroxypyrazol in 30 ml Ethanol getropft. Man rührt zwei Stunden bei Raumtemperatur und engt das Reaktionsgemisch ein. Der Rückstand wird in 60 ml Dimethylformamid aufgenommen und mit 5,7 g (0,02 mol) α-(2-Brommethylphenyl)-β-methoxy-acrylsäure-methylester in 30 ml Dimethylformamid versetzt. Man rührt zwei Stunden bei 100 °C, zieht das Lösungsmittel ab und nimmt den Rückstand in Methyl-tert.-butylether auf. Die organische Phase wird mit Wasser gewaschen, getrocknet und eingeengt. Man erhält 5,0 g (77 %) der Titelverbindung als Öl (Verbindung Nr. 9).

In entsprechender Weise werden die folgenden Verbindungen hergestellt.

Tabelle 1: Verbindungen der Formel I

Die Konfigurationsangabe bezieht sich auf die β-Methoxy-acrylsäuremethylestergruppe bzw. auf die Glyoxylsäure-methylester-O-methyloximgruppe.

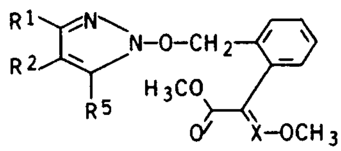

I

| Nr. | $R^1$ | $R^2$ | $R^3$ | X | Fp. (°C) |
|---|---|---|---|---|---|
| 1 | H | H | H | CH | öl (E) |
| 2 | H | H | H | N | öl (E) |
| 3 | H | $CH_3$ | H | CH | |
| 4 | H | $CH_3$ | H | N | |
| 5 | H | $C_2H_5$ | H | CH | |
| 6 | H | $C_2H_5$ | H | N | |
| 7 | H | F | H | CH | |
| 8 | H | F | H | N | |
| 9 | H | Cl | H | CH | öl (E) |
| 10 | H | Cl | H | N | öl (E) |
| 11 | H | Br | H | CH | |
| 12 | H | Br | H | N | |
| 13 | H | $C_6H_5$ | H | CH | |
| 14 | H | $C_6H_5$ | H | N | |
| 15 | H | $2-Cl-C_6H_4$ | H | CH | |
| 16 | H | $2-Cl-C_6H_4$ | H | N | |
| 17 | H | $4-Cl-C_6H_4$ | H | CH | |
| 18 | H | $4-Cl-C_6H_4$ | H | N | |
| 19 | H | $2-CH_3-C_6H_4$ | H | CH | |
| 20 | H | $2-CH_3-C_6H_4$ | H | N | |
| 21 | H | $4-CH_3-C_6H_4$ | H | CH | |
| 22 | H | $4-CH_3-C_6H_4$ | H | N | |
| 23 | H | $C_6H_5-CH_2$ | H | CN | |
| 24 | H | $C_6H_5-CH_2$ | H | N | |
| 25 | H | $CO_2CH_3$ | H | CH | |
| 26 | H | $CO_2CH_3$ | H | N | |
| 27 | $CH_3$ | H | $CH_3$ | CH | öl (E) |
| 28 | $CH_3$ | H | $CH_3$ | N | öl (E) |
| 29 | $C_2H_5$ | H | $C_2H_5$ | CH | |
| 30 | $C_2H_5$ | H | $C_2H_5$ | N | |
| 31 | F | H | F | CH | |

Tabelle 1 (Fortsetzung)

| Nr. | $R^1$ | $R^2$ | $R^3$ | X | Fp. (°C) |
|---|---|---|---|---|---|
| 32 | F | H | F | N | |
| 33 | Cl | H | Cl | CH | |
| 34 | Cl | H | Cl | N | |
| 35 | Br | H | Br | CH | |
| 36 | Br | H | Br | N | |
| 37 | $C_6H_5$ | H | $C_6H_5$ | CH | |
| 38 | $C_6H_5$ | H | $C_6H_5$ | N | |
| 39 | $C_6H_5-CH_2$ | H | $C_6H_5-CH_2$ | CH | |
| 40 | $C_6H_5-CH_2$ | H | $C_6H_5-CH_2$ | N | |
| 41 | $CH_3$ | Cl | $CH_3$ | CH | |
| 42 | $CH_3$ | Cl | $CH_3$ | N | |
| 43 | $CH_3$ | Br | $CH_3$ | CH | |
| 44 | $CH_3$ | Br | $CH_3$ | N | |
| 45 | $C_2H_5$ | Cl | $C_2H_5$ | CH | |
| 46 | $C_2H_5$ | Cl | $C_2H_5$ | N | |
| 47 | $C_2H_5$ | Br | $C_2H_5$ | CH | |
| 48 | $C_2H_5$ | Br | $C_2H_5$ | N | |
| 49 | $CH_3$ | $CO_2CH_3$ | $CH_3$ | CH | |
| 50 | $CH_3$ | $CO_2CH_3$ | $CH_3$ | N | |
| 51 | H | A | A | CH | |
| 52 | H | A | A | N | |
| 53 | H | B | B | CH | |
| 54 | H | B | B | N | |

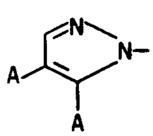 = 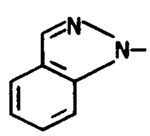

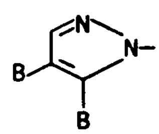 = 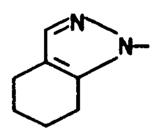

Tabelle 2:

Spektroskopische Daten ($^1H$-NMR und IR) ausgewählter Verbindungen aus der Tabelle 1.

Bei den NMR-Spektren wird die chemische Verschiebung (δ) in ppm relativ zu Tetramethylsilan angegeben. Als Lösungsmittel dient $CDCl_3$.

Bei den IR-Spektren werden die Wellenzahlen in $cm^{-1}$ angegeben.

Verbindung Nr. 1

NMR:
3,67 (s, 3H); 3,73 (s, 3H); 5,17 (s, 2H); 5,96 (m, 1H); 6,96 (m, 1H); 7,16-7,37 (m, 5H); 7,60 (s, 1H).

IR (Film):
1706, 1635, 1436, 1286, 1258, 1208, 1192, 1131, 1111, 748.

Verbindung Nr. 2

NMR:
3,87 (s, 3H); 4,04 (s, 3H); 5,13 (s, 2H); 5,98 (m, 1H); 7,00 (m, 1H); 7,17-7,45 (m, 5H).

IR (Film):
1726, 1438, 1323, 1224, 1201, 1069, 1046, 1019, 963, 745.

Verbindung Nr. 9

NMR:
3,69 (s, 3H); 3,79 (s, 3H); 5,15 (s, 2H); 6,96 (s, 1H); 7,13-7,40 (m, 5H); 7,60 (s, 1H).

IR (Film):
1706, 1636, 1286, 1258, 1209, 1191, 1131, 1111, 967, 770.

Verbindung Nr. 10

NMR:
3,88 (s, 3H); 4,04, (s, 3H); 5,13 (s, 2H); 7,01 (s, 1H); 7,16 (s, 1H); 7,20-7,49 (m, 4H).

IR (Film):
1726, 1438, 1317, 1223, 1203, 1069, 1019, 982, 967, 768.

Verbindung Nr. 27

NMR:
1,89 (s, 3H); 2,20 (s, 3H); 3,68 (s, 3H); 3,73 (s, 3H); 5,13 (s, 2H); 5,63 (s, 1H); 7,13-7,39 (m, 4H); 7,60 (s, 1H).

IR (Film):
1709, 1634, 1435, 1285, 1258, 1207, 1191, 1131, 1112, 770.

Verbindung Nr. 28

NMR:
1,88 (s, 3H); 2,20 (s, 3H); 3,83 (s, 3H); 4,02 (s, 3H); 5,08 (s, 2H); 5,63 (s, 1H); 7,20-7,43 (m, 4H).

IR (Film):
1728, 1438, 1321, 1223, 1201, 1069, 1019, 959, 779, 770.

Die neuen Verbindungen zeichnen sich, allgemein ausgedrückt, durch eine hervorragende Wirksamkeit gegen ein breites Spektrum von pflanzenpathogenen Pilzen, insbesondere aus der Kiasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,

Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. PolyoxyethylenFettalkohol-Ether,Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 9 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 27 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 9 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 27 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 9 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 27 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe 2, 9 und 27 bei der Anwendung als 0,05 %ige (Gew.%) Spritzbrühe eine gute fungizide Wirkung zeigen (95 %).

**Patentansprüche**

**1.** Substituierte N-Hydroxypyrazole der allgemeinen Formel I

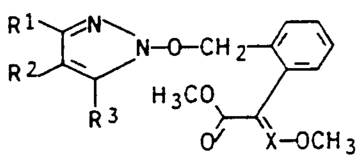

I

in der
$R^1$, $R^2$ und $R^3$ gleich oder verschieden sind und Wasserstoff, $C_1$-$C_4$-Alkyl, $C_1$-$C_2$-Halogenalkyl, $C_3$-$C_6$-Cycloalkyl, $C_1$-$C_4$-Alkoxycarbonyl, Halogen, Phenyl oder Phenyl-$C_1$-$C_4$-alkyl bedeuten, wobei der Phenylrest gegebenenfalls durch einen oder mehrere der folgenden Reste substituiert ist: $C_1$-$C_4$-Alkyl, $C_3$-$C_6$-Cycloalkyl, $C_1$-$C_2$-Halogenalkyl, $C_1$-$C_4$-Alkoxy, Halogen, Cyano, Nitro,
oder $R^2$ und $R^3$ zusammen mit dem Pyrazolring, dessen Substituenten sie sind, einen Indazolring oder Tetrahydroindazolring bilden, wobei diese Ringe gegebenenfalls einfach bis dreifach durch $C_1$-$C_4$-Alkyl substituiert sind,
X CH oder N bedeutet und ihre pflanzenverträglichen Säureadditionssalze und Metallkomplexe.

**2.** Verfahren zur Herstellung substituierter N-Hydroxypyrazole der allgemeinen Formel I gemäß Anspruch 1, dadurch gekennzeichnet, daß man ein N-Hydroxypyrazol der allgemeinen Formel II,

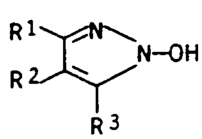

II

in der
$R^1$, $R^2$ und $R^3$ die im Anspruch 1 genannten Bedeutungen haben mit einer substituierten Benzylverbindung der allgemeinen Formel III

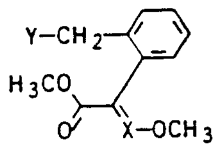

III

in der
X CH oder N und
Y Chlorid, Bromid, p-Toluolsulfonat, Methansulfonat oder Trifluormethansulfonat bedeutet, in einem Lösungs- oder Verdünnungsmittel in Gegenwart einer Base und gegebenenfalls eines Katalysators umsetzt.

**3.** Fungizid, enthaltend einen inerten Trägerstoff und eine fungizid wirksame Menge eines substituierten N-Hydroxypyrazols der allgemeinen Formel I gemäß Anspruch 1.

**4.** Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines substituierten N-Hydroxypyrazols der allgemeinen Formel I gemäß Anspruch 1 behandelt.

**5.** Verbindung der Formel I gemäß Anspruch 1, in der $R^1$ und $R^3$ Wasserstoff, $R^2$ Chlor und X den Rest CH bedeutet.

**6.** Verbindung der Formel I gemäß Anspruch 1, in der $R^1$ und $R^3$ Wasserstoff, $R^2$ Chlor und X Stickstoff bedeutet.

**7.** Verbindung der Formel I gemäß Anspruch 1, in der $R^1$ und $R^3$ Methyl, $R^2$ Wasserstoff und X den Rest CH bedeutet.

**8.** Verbindung der Formel I gemäß Anspruch 1, in der $R^1$ und $R^3$ Methyl, $R^2$ Wasserstoff und X Stickstoff bedeutet.

**9.** Verbindung der Formel I gemäß Anspruch 1, in der $R^1$, $R^2$ und $R^3$ Wasserstoff und X Stickstoff bedeutet.

## Claims

**1.** A substituted N-hydroxypyrazole of the general formula I

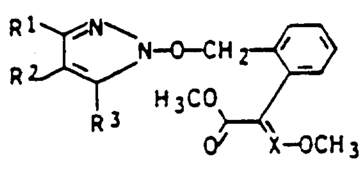

I

where $R^1$, $R^2$ and $R^3$ are identical or different and are each hydrogen, $C_1$-$C_4$-alkyl, $C_1$-$C_2$-haloalkyl, $C_3$-$C_6$-cycloalkyl, $C_1$-$C_4$-alkoxycarbonyl, halogen, phenyl or phenyl-$C_1$-$C_4$-alkyl, the phenyl radical being unsubstituted or substituted by one or more of the following: $C_1$-$C_4$-alkyl, $C_3$-$C_6$-cycloalkyl, $C_1$-$C_2$-haloalkyl, $C_1$-$C_4$-alkoxy, halogen, cyano or nitro
or $R^2$ and $R^3$ form, together with the pyrazole ring of which they are substituents, an indazole ring or tetrahydroindazole ring, these rings being unsubstituted or substituted one to three times by $C_1$-$C_4$-alkyl,
X is CH or N, or an acid addition salt or metal complex thereof which is tolerated by plants.

2. A process for preparing a substituted N-hydroxypyrazole of the general formula I as claimed in claim 1, which comprises reacting an N-hydroxypyrazole of the general formula II

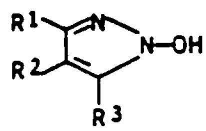

II

where $R^1$, $R^2$ and $R^3$ have the meanings stated in claim 1, with a substituted benzyl compound of the general formula III

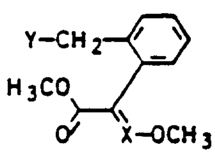

III

where X is CH or N, and Y is chloride, bromide, p-toluenesulfonate, methanesulfonate or trifluoromethanesulfonate, in a solvent or diluent in the presence of a base and in the presence or absence of a catalyst.

3. A fungicide containing an inert carrier and a fungicidally effective amount of a substituted N-hydroxypyrazole of the general formula I as claimed in claim 1.

4. A method for controlling fungi, wherein the fungi, or the materials, plants, seed or soil threatened by fungi attack, are treated with a fungicidally effective amount of a substituted N-hydroxypyrazole of the general formula I as claimed in claim 1.

5. A compound of the formula I as claimed in claim 1, where $R^1$ and $R^3$ are each hydrogen, $R^2$ is chlorine and X is CH.

6. A compound of the formula I as claimed in claim 1, where $R^1$ and $R^3$ are each hydrogen, $R^2$ is chlorine and X is nitrogen.

7. A compound of the formula I as claimed in claim 1, where $R^1$ and $R^3$ are each methyl, $R^2$ is hydrogen and X is CH.

8. A compound of the formula I as claimed in claim 1, where $R^1$ and $R^3$ are each methyl, $R^2$ is hydrogen and X is nitrogen.

9. A compound of the formula I as claimed in claim 1, where $R^1$, $R^2$ and $R^3$ are each hydrogen and X is nitrogen.

**Revendications**

1. N-Hydroxypyrazoles substitués, de formule générale I

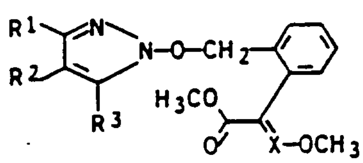

I

dans laquelle
$R^1$, $R^2$ et $R^3$ sont identiques ou différents et représentent hydrogène, alkyle en C1-C4, halogénalkyle en C1-C2, cycloalkyle en C3-C6, alcoxy en C1-C4-carbonyle, halogène, phényle ou phényl-alkyle en C1-C4 le reste phényle étant éventuellement substitué par un ou plusieurs restes, à savoir alkyle en C1-C4, cycloalkyle en C3-C6, halogénalkyle en C1-C2, alcoxy en C1-C4, halogène, cyano, nitro,
ou $R^2$ et $R^3$, ensemble avec le cycle pyrazole dont ils sont les substituants, formant un cycle indazole ou tétrahydro-indazole, ces cycles étant éventuellement substitués une à trois fois par alkyle en C1-C4,
X représente CH ou N,
ainsi que leurs sels d'addition d'acide et leurs complexes métalliques tolérés par les plantes.

**2.** Procédé de préparation de N-hydroxypyrazoles substitués, de formule générale I, selon la revendication 1, caractérisé par le fait que l'on fait réagir, dans un solvant ou un diluant, en présence d'une base et éventuellement d'un catalyseur, un N-hydroxypyrazole de formule générale II

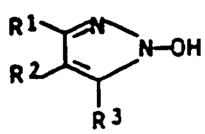

II

dans laquelle
$R^1$, $R^2$ et $R^3$ ont les significations données dans la revendication 1, avec un dérivé de benzyle substitué, de formule générale III

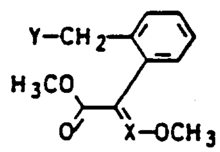

III

dans laquelle
X = CH ou N et
Y = chlorure, bromure, p-toluènesulfonate, méthanesulfonate ou trifluorométhanesulfonate.

**3.** Fongicide, contenant un support inerte et une quantité efficace au point de vue fongicide d'un N-hydroxypyrazole substitué, de formule générale I, selon la revendication 1.

**4.** Procédé de lutte contre les champignons, caractérisé par le fait que l'on traite les champignons ou les matériaux, plantes, semences ou le sol avec une quantité efficace au point de vue fongicide d'un N-hydroxypyrazole substitué, de formule générale I, selon la revendication 1.

**5.** Composé de formule I, selon la revendication 1, dans lequel $R^1$ et $R^3$ représentent hydrogène, $R^2$ chlore et X le reste CH.

**6.** Composé de formule I, selon la revendication 1, dans lequel $R^1$ et $R^3$ représentent hydrogène, $R^2$ chlore et X azote.

**7.** Composé de formule I, selon la revendication 1, dans lequel $R^1$ et $R^3$ représentent méthyle, $R^2$ hydrogène et X le reste CH.

**8.** Composé de formule I, selon la revendication 1, dans lequel $R^1$ et $R^3$ représentent méthyle, $R^2$ hydrogène et X azote.

**9.** Composé de formule I, selon la revendication 1, dans lequel $R^1$, $R^2$ et $R^3$ représentent hydrogène et X azote.